Europäisches Patentamt

European Patent Office

Office européen des brevets

(11) Veröffentlichungsnummer: **0 005 807**
A1

## (12) EUROPÄISCHE PATENTANMELDUNG

(21) Anmeldenummer: 79101608.2

(22) Anmeldetag: 25.05.79

(51) Int. Cl.²: **A 61 K 7/06**
A 61 K 7/08, A 61 K 7/11

(30) Priorität: 01.06.78 DE 2824025

(43) Veröffentlichungstag der Anmeldung:
12.12.79 Patentblatt 79 25

(84) Benannte Vertragsstaaten:
BE CH FR GB IT NL SE

(71) Anmelder: Henkel Kommanditgesellschaft auf Aktien
Postfach 1100
D-4000 Düsseldorf 1(DE)

(72) Erfinder: Cortekar, Hans-Wolfgang
Elisabethstrasse 38
D-4018 Langenfeld-Richrath(DE)

(54) Verwendung reduzierend wirkender Zucker zur Verbesserung der Struktur und der Nasskämmbarkeit von Haaren.

(57) Gegenstand der Erfindung ist die Verwendung reduzierend wirkender Zucker in Haarkonditionierungsmitteln zur gleichzeitigen Verbesserung der Struktur und der Naßkämmbarkeit von Haaren.

EP 0 005 807 A1

Croydon Printing Company Ltd.

0005807

HENKEL KGaA
ZR-FE/Patente
z-sü

4000 Düsseldorf, den
Henkelstraße 67

P a t e n t a n m e l d u n g

D 5759

"Verwendung reduzierend wirkender Zucker zur Verbesserung der Struktur und der Naßkämmbarkeit von Haaren"

Gegenstand der Erfindung ist die Verbesserung der Struktur und der Naßkämmbarkeit von Haaren, insbesondere von
Haaren, die durch haarkosmetische Behandlungen wie
Färbungen, Entfärbungen, Dauerwellen nachteilig beeinflußt wurden durch die Behandlung mit haarkosmetischen
Mitteln, die reduzierend wirkende Zucker enthalten.

Als Folge der häufigen Behandlung des Haares mit mehr
oder weniger stark schädigenden Mitteln beim Bleichen,
Dauerwellen und gegebenenfalls auch Färben tritt neben
einer Strukturschädigung auch eine zunehmende Verwirrung
der Haare ein, die vor allem bei vollem Haar zu einer
wesentlichen Verschlechterung der Naßkämmbarkeit führt.
Darüber hinaus bekommt das Haar vielfach ein stumpfes,
glanzloses Aussehen und einen verschlechterten Griff.

/2

0005807

Um diesem Übelstand zu begegnen, hat man den haarkosmetischen Präparaten bereits strukturverbessernde Mittel und Produkte zur Beeinflussung der Naßkämmbarkeit zugesetzt. Als Mittel zur Verbesserung der Haarstruktur wurden in erster Linie Formaldehyd sowie formaldehydabspaltende Verbindungen, ferner S-Acetylsuccinanhydrid, Ammoniumvinylphosphonat und andere eingesetzt. Der Verbesserung der Kämmbarkeit diente in erster Linie der Zusatz von Fetten, höheren Kohlenwasserstoffen in Frisiercremes und Brillantinen und von quartären Ammoniumverbindungen. Um sowohl eine strukturverbessernde Wirkung als auch eine verbesserte Naßkämmbarkeit zu erreichen, bedurfte es des Zusatzes mehrerer Mittel, da mit einem Produkt eine Beeinflussung in beiden Richtungen nicht zu erreichen war.

Es wurde nun gefunden, daß sowohl eine strukturverbessernde Wirkung als auch eine verbesserte Naßkämmbarkeit zu erzielen ist, wenn man in den haarkosmetischen Mitteln reduzierend wirkende Zucker einsetzt.

Als reduzierende Zucker können in den haarkosmetischen Zubereitungen erfindungsgemäß Glucose, Fructose, Invertzucker, Galaktose, Mannose, Maltose, Lactose eingesetzt werden, wobei der Glucose die größte Bedeutung zukommt.

Der Einsatz der reduzierenden Zucker kann in Frisierlotionen, Frisiercremes, Frisiergelen, Antischuppenlotionen, Wasserwellmitteln, Haarspülungen, Tönungswäschen, Fönwellen und ähnlichen Mitteln erfolgen.

Durch die Behandlung des Haares mit den erfindungsgemäßen, reduzierende Zucker enthaltenden Mitteln wird erreicht, daß sich das feuchte Haar wesentlich leichter kämmen läßt. Darüber hinaus zeichnet sich das trockene Haar durch einen besseren Glanz, Fülle, angenehmen    /3 Griff aus und zeigt keine Neigung zur statischen Aufladung beim Bürsten oder Kämmen. Dies gilt sowohl für normales Haar als auch insbesondere für Haar, das einer Behandlung und schädigenden Mitteln beim Bleichen, Dauerwellen oder Färben ausgesetzt worden ist.

Die erfindungsgemäß in den haarkosmetischen Mitteln zu verwendenden reduzierenden Zucker können in Mengen von 0,5 bis 8 Gewichtsprozent, vorzugsweise von 1 - 5 Gewichtsprozent, bezogen auf das gesamte Mittel, eingesetzt werden. Daneben können die Mittel grenzflächenaktive Verbindungen, Verdickungsmittel, Kunstharze, Weichmacher, Antischuppenmittel, Konservierungsstoffe, Parfüms, Lösungsmittel wie niedere Alkohole und andere übliche Rezepturbestandteile enthalten. Die haarkosmetischen Mittel können als Cremes, Gele, Lotionen und in seltenen Fällen auch als Aerosole mit einem Treibmittelanteil vorliegen.

Die nachfolgenden Beispiele sollen den Erfindungsgegenstand näher erläutern, ohne ihn jedoch hierauf zu beschränken.

/4

## B e i s p i e l e

1) Haarspülbad

| | |
|---|---|
| Kolloiddisperses Gemisch von Cetylstearylalkohol, Natriumcetylstearylsulfat und nichtionogenen Emulgator, Emulgade F$^{(R)}$ Dehydag | 3,5 Gew.-Teile |
| Ölsäuredecylester | 10,0 " |
| Glycerin | 3,0 " |
| Glucose | 2,0 " |
| Zitronensäure | 3,0 " |
| Parfüm | 0,5 " |
| Wasser | 78,0 " |

2) Haarregenerator

| | |
|---|---|
| Cetylstearylalkohol | 4,0 Gew.-Teile |
| Glucose | 4,0 " |
| Zitronensäure | 1,0 " |
| Copaiva Balsamöl | 0,1 " |
| Parfüm | 1,0 " |
| Wasser | 89,9 " |

3) Haarfestiger

| | |
|---|---|
| Vinylpyrrolidon/Vinylacetat-Copolymeres Nasuna B$^{(R)}$, Dehydag | 1,5 Gew.-Teile |
| Glucose | 1,5 " |
| Isopropanol | 50,0 " |
| Parfüm | 1,0 " |
| Wasser | 46,0 " |

/5

0005807

4) Birkenhaarwasser

| | | |
|---|---|---|
| Polyol-Fettsäureester, Cetiol HE$^{(R)}$ Dehydag | 2,0 | Gew.-Teile |
| Extrapon Birke spezial | 1,0 | " |
| Glucose | 3,0 | " |
| Isopropanol | 30,0 | " |
| Parfüm | 1,0 | " |
| Wasser | 63,0 | " |

5) Haarwasser

| | | |
|---|---|---|
| Isopropanol | 60,0 | Gew.-Teile |
| Glycerin | 3,0 | " |
| Glucose | 2,0 | " |
| 2,2'-Dioxy-3,5,6,3',5',6'-hexachlordiphenylmethan | 0,2 | " |
| Parfüm | 1,0 | " |
| Wasser | 33,8 | " |

6) Frisiercreme

| | | |
|---|---|---|
| Polyolfettsäureester | 8,0 | Gew.-Teile |
| Isopropanol | 20,0 | " |
| Verdickungsmittel Carbopol 940 | 1,0 | " |
| Triäthanolamin | 0,8 | " |
| Glucose | 4,0 | " |
| Parfüm | 1,2 | " |
| Wasser | 65,0 | " |

An die Stelle von Glucose können in vorstehenden Beispielen auch die anderen genannten reduzierenden Zucker treten.

0005807

HENKEL KGaA
ZR-FE/Patente

"Verwendung reduzierend wirkender Zucker zur Verbesserung der Struktur und der Naßkämmbarkeit von Haaren"

Patentansprüche:

1. Verwendung von reduzierend wirkenden Zuckern in haarkosmetischen Mitteln zur gleichzeitigen Verbesserung der Struktur und der Naßkämmbarkeit von Haaren.

2. Verwendung von Glucose, Fructose, Invertzucker, Galaktose, Mannose, Maltose, Lactose nach Anspruch 1.

3. Verwendung von Glucose nach Anspruch 1 und 2.

4. Verwendung von reduzierend wirkenden Zuckern nach Anspruch 1 - 3, in einer Menge von 0,5 bis 8 Gewichtsprozent, vorzugsweise 1 bis 5 Gewichtsprozent, bezogen auf das gesamte haarkosmetische Mittel.

| | | Nummer der Anmeldung |
|---|---|---|
| ◉ Europäisches Patentamt | **EUROPÄISCHER RECHERCHENBERICHT** | EP 79 10 1608 |

| **EINSCHLÄGIGE DOKUMENTE** | | | **KLASSIFIKATION DER ANMELDUNG (Int.Cl.²)** |
|---|---|---|---|
| Kategorie | Kennzeichnung des Dokuments mit Angabe, soweit erforderlich, der maßgeblichen Teile | betrifft Anspruch | |

| Kategorie | Dokument | betrifft Anspruch |
|---|---|---|
| X | CH - A - 443 567 (COLGATE PALMO-LIVE) <br> * Spalte 1, Zeilen 29-31; Spalte 3, Zeilen 14-36; Beispiele 1, 7-10,13; Ansprüche und Unteransprüche 1 und 3 * <br> -- | 1-4 |
| X | CHEMICAL ABSTRACTS, Band 82, Nr. 10, 10. März 1975, Nr. 64326m Columbus, Ohio, U.S.A. <br> I. SCHRECK-PUROLA et al.: "Composition for preserving and renewing the hair", Seite 460, Spalte 1, Zusammenfassung 64326m <br> & SF - C - 48 532 (I. SCHRECK-PUROLA et al.) 31-07-1974 <br> * Zusammenfassung * <br> -- | 1-4 |
| | US - A - 2 383 990 (S. QUISLING) <br> * Seite 1, linke Spalte, Zeilen 1-6; Seite 1, rechte Spalte, Zeilen 35-40; Seite 2, linke Spalte, Zeile 45 * <br> -- | 1,2 |
| | DE - A - 2 141 764 (HENKEL) <br> * Seite 1, Zeilen 1-4; Seite 4, Zeilen 10-19 * <br> -- | 1-4 |
| | DE - A - 2 141 763 (HENKEL) <br> * Seite 5, Zeilen 7-19 * <br> -- | 1-4 |
| | DE - C - 905 193 (HENKEL) <br> * Seite 1, Zeilen 1-7; Beispiel; | 1,2 |

**KLASSIFIKATION DER ANMELDUNG (Int.Cl.²)**

A 61 K 7/06
7/08
7/11

**RECHERCHIERTE SACHGEBIETE (Int. Cl.²)**

A 61 K 7/06
7/08
7/09
7/11

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| | Der vorliegende Recherchenbericht wurde für alle Patentansprüche erstellt. |
|---|---|

| Recherchenort | Abschlußdatum der Recherche | Prüfer |
|---|---|---|
| Den Haag | 06-09-1979 | WILLEKENS |

EPA form 1503.1 06.78

# EUROPÄISCHER RECHERCHENBERICHT

Europäisches
Patentamt

Nummer der Anmeldung

EP 79 10 1608

-2-

| | EINSCHLÄGIGE DOKUMENTE | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | Anspruch 1 * | | |
| | -- | | |
| | GB - A - 552 285 (N.H. CHAMBER-LAIN) | 1-3 | |
| | * Beispiel * | | |
| | ---- | | |

RECHERCHIERTE SACHGEBIETE (Int. Cl.²)